Europäisches Patentamt

(19) European Patent Office

Office européen des brevets

(11) Publication number: **0 077 664**
**B1**

(12) **EUROPEAN PATENT SPECIFICATION**

(45) Date of publication of patent specification: **16.05.90**

(21) Application number: **82305508.2**

(22) Date of filing: **15.10.82**

(51) Int. Cl.⁵: **C 12 N 15/00,** C 12 N 9/52,
C 12 N 1/20 // C12R1/19

(54) **Recombinant DNA and coliform bacillus having genetic material for the production of staphylokinase, and the production of staphylokinase.**

(30) Priority: **16.10.81 JP 164064/81**

(43) Date of publication of application:
**27.04.83 Bulletin 83/17**

(45) Publication of the grant of the patent:
**16.05.90 Bulletin 90/20**

(84) Designated Contracting States:
**BE CH DE FR GB IT LI NL SE**

(56) References cited:
**BIORESEARCH INDEX, vol. 24, 1983, ref. 70921, International Union of Microbiological Societies. 13th International Congress of Microbiology, Boston, USA, 8th-13th August 1982, National Technical Information Service, Springfield, USA**
**CHEMICAL ABSTRACTS, vol. 93, no. 9, 1st September 1980, page 331, no. 91712t, Columbus, Ohio, USA**
**INFECTION AND IMMUNITY, vol. 18, no. 2, November 1977, pages 266-272, USA**
**PROC. NATL. ACAD. SCI. USA, vol. 78, no. 6, June 1981, pages 3313-3317, USA**

(73) Proprietor: **KABUSHIKI KAISHA YAKULT HONSHA**
**1-19, Higashishinbashi 1-chome**
**Minato-ku Tokyo 105 (JP)**

(72) Inventor: **Sako, Tomoyuki**
**3-802, 3811, Haejimacho**
**Akishima-shi Tokyo (JP)**
Inventor: **Sawaki, Saeko**
**4-15-9, Higashi**
**Kunitachi-shi Tokyo (JP)**
Inventor: **Sakurai, Toshizo**
**2-7-19, Nakahara**
**Mitaki-shi Tokyo (JP)**
Inventor: **Mutai, Masahiko**
**4-988, Shimizu**
**Higashiyamato-shi Tokyo (JP)**
Inventor: **Kondo, Isamu**
**2-12-10, Yushima**
**Bunkyo-ku Tokyo (JP)**

(74) Representative: **Bizley, Richard Edward et al**
**BOULT, WADE & TENNANT 27 Furnival Street**
**London EC4A 1PQ (GB)**

Courier Press, Leamington Spa, England.

**Description**

This invention relates to recombinant DNA and a coliform bacillus having genetic material encoded for staphylokinase production (which material is hereinafter referred to as "SAK gene"), and to a process for the production of staphylokinase by cultivating the bacillus.

Staphylokinase (hereinafter referred to as "SAK") is a fibrinolytic enzyme which is produced by Staphylococcus aureus (hereinafter referred to as "S. aureus").

The function of SAK is to transform plasminogen in blood into plasmin, which in turn acts on fibrin to lyse the same. There are known, as enzymes of similar activity, urokinase present in human urine and streptokinase produced by streptococci. All these enzymes find use in medicine as fibrinolytics, for example, anticoagulants (blood coagulation inhibitors) and antithrombotics. In view of such usefulness, many attempts have been made to improve processes for the production, extraction and purification of these enzymes.

As for the production of SAK, it is known that the capability of producing SAK is a trait imparted to a S. aureus host by way of what is called lysogenic conversion, i.e. lysogenization in the host involving a temperate phage. Accordingly, prior art processes for the production of SAK comprise selecting from strains of S. aureus a strain having SAK producing ability, or lysogenizing a S. aureus strain incapable of SAK production with a phage capable of lysogenic conversion of SAK, culturing, in either case, the thus-obtained strain and obtaining SAK from the culture.

S. aureus is, however, a bacterium exhibiting strong pathogenicity, and so in the handling thereof great caution is required. Therefore the prior art processes are not suited for SAK production on an industrial scale. Moreover, in order to achieve high yields of SAK, it is necessary to carry out cultivation for a long period of time which in inappropriate for SAK production on an industrial scale.

This invention is based upon the discovery of a process which is suitable, from the standpoints of both safety and efficiency, for SAK production on an industrial scale. We have now found that the gene coding for SAK production is located on phage DNAs and have succeeded in introducing, through a vector into E. coli, a SAK gene-carrying fragment of a temperate phage of S. aureus.

Thus according to the present invention there is provided recombinant DNA carrying genetic material encoded for the production of staphylokinase and derived from a temperate phage of Staphylococcus aureus, said DNA being obtainable from ATCC 39179 or ATCC 39178 in the form of a sequence 4.9 kb in size by digestion with Hind III, or DNA being DNA exhibiting at least partial homology to said sequence, in either case the DNA being capable of transforming a host coliform bacillus to introduce said genetic material into the host genotype whereby said genetic material may be phenotypically expressed by the host to produce staphylokinase.

The invention further provides a coliform bacillus having as part of its phenotypically expressible genotype recombinant DNA of the invention.

The novel bacillus according to the present invention can be obtained by a process which comprises:—
subjecting a temperate phage DNA of Staphyloccus aureus to restriction enzyme digestion to produce DNA fragments, optionally isolating the DNA fragment which is encoded for staphylokinase production, cleaving a DNA vector capable of transforming a host coliform bacillus with one or more restriction enzymes, ligating said fragment or fragments into resulting vector cleavage site(s), introducing the resulting recombinant DNA into a coliform bacillus host, and selectively separating those of the resulting coliform bacillus cells which have thereby gained the capability of producing staphylokinase.

As the temperate phage which serves as the original DNA donor, any strain may be employed which is separated from a lysogenic strain of S. aureus and which is capable of lysogenic conversion. Examples of such phage strains include 42D, L42E and 77 (Mason, R.E. and Allen, W.E. (1975) Can. J. Microbiol. *21*, 1113—1116); Pφ1, Pφ2, Tφ-42D and Pφ-406 (Kondo, I. and Fujise, K. (1977) Infect. Immun. *18*, 266—272); Rφ19 and SφC (Kondo, I. et al (1980) Tokyo Jikei-kai Ika-Daigaku Zasshi *95*, 1203—1206).

Preparation of temperate phage DNAs may be carried out by conventional methods in the art, which comprise infecting a strain of S. aureus as the host with a temperate phage strain and culturing the thus-infected microbe (see, for example, Blair, J.E. and Williams, R.E.O. (1961) Bull, W.H.O. *24*, 771—784). As a result of the cultivation, the cells are lysed and the phage is released into the culture medium. The phage thus released is collected by an appropriate method (for example by CsCl equilibrium density gradient centrifugation as described in Rosenblum, E.D. and Tyrone, S. (1964) J. Bacteriol. *88*, 1737—1742). Isolation of the phage DNA from the thus collected phage may be carried out by methods known *per se* in the art, for example, by extraction with phenol.

Digestion of temperate phage DNAs with one or more restriction enzymes may be carried out in the following manner.

An appropriate restriction enzyme is added to the phage DNA and brought into reaction therewith under appropriate conditions, with the result that the phage DNA is digested into fragments which vary with the restriction enzyme used.

Restriction enzymes suitable for digesting the phage DNA must (1) be able to excise the phage DNA and (2) not excise the DNA region carrying genetic information encoded for SAK production. Preferred examples of such restriction enzymes include Hind III, Pst I, Ava II, EcoR I, Hpa I, Hpa II, Hind II, Sst I, Cla I, BstE II, Sst II, Xho I, Bcl I, Bgl II, Pvu II, X or II, Kpn I, Sma I and Xba I.

2

After the digestion, the DNA fragment carrying genetic information for SAK production may be isolated from the restriction fragments of the phage DNA. The isolation may be carried out by means known *per se* in the art, for example by agarose gel electrophoresis. In carrying out the isolation, it is necessary to determine beforehand which one of the restriction fragments from the phage DNA carries genetic information encoded for production of SAK. Reference to the method for the such determination will be made in detail later. As mentioned already, this isolation step may be omitted if desired or necessary.

Cleavage of vector DNAs can be carried out by adding an appropriate restriction enzyme to the vector DNA and bringing them into reaction under appropriate conditions.

For the purpose of the present invention any vector DNA known in the art may be used. Preferred examples of vector DNAs include Col E1, pMB 9, pSC 101 and p15A and their derivatives such as pBR 322 and pACYC 184, as well as Charon vectors derived from λ phage.

Insertion of the above-mentioned SAK gene-carrying DNA fragment (or fragments is the isolation step has been omitted) into the vector DNA at the site of cleavage of the latter can be carried out by conventional means. Suitable reaction conditions are chosen dependent upon the particular phage DNA, vector DNA and restriction enzyme(s) used.

The insertion of the phage DNA fragment(s) into the vector DNA may take place in any orientation and at any site so long as the vector's capability of replication is not impaired.

The recombinant DNA produced by the above procedure is an entirely new and extremely useful product which is included in the present invention.

The recombinant DNA may then be introduced into an E. coli host. All E. coli strains are usable as the E. coli host whose restriction-modification system is devoid of the capability of restriction. Modified plasmids in accordance with the invention into which has been inserted DNA carrying genetic information encoded for the production of SAK may also be used for E. coli strains having the capability of restriction. The range of possible types of E. coli host may however, be limited depending upon the kind of vector used. In the designation of the E. coli strains used, "$r_K^-$" means that the strain is devoid of the capability of restriction and "$m_K^-$" that the strain is devoid of the capability of modification and "$m_K^+$" that the strain has the capability of modification.

The introduction of the recombinant DNA can be carried out by means and techniques known *per se*, as described, for example, in Cameron, et al. (1975) Proc. Natl. Acad. Sci., U.S.A. *72*, 3416—3420.

Even when the fragment bearing the SAK gene is isolated and ligation thereof into the vector cleavage site is attempted, it sometimes happens that no insertion of the SAK gene-carrying DNA fragment of temperate phages takes place. Furthermore, when such isolation is omitted there is the possibility, in addition to the possibility of no insertion taking place, that other DNA fragments not relevant to SAK production are inserted. In order to overcome these problems it is necessary to effect a selective separation of E. coli cells which have gained the capability of producing SAK. For this purpose it is convenient to employ the procedure known for S. aureus, as described in Kondo, I. and Fujise, K. (1977) Infect. Immun. *18*, 266—272. Thus, in accordance with the known procedure, heated plasma agar medium is prepared and the microbe to be tested spotted thereupon and grown for a given period of time to examine the fibrinolytic activity. The microbes having fibrinolytic activity form transparent lytic halos around their colonies, from which the desired microbe can be isolated. In this way the desired SAK-producing E. coli strains can be isolated.

Anlysis of the SAK gene-carrying DNA from the SAK-producing E. coli can be carried out in the following manner. Thus, the recombinant plasmid or the recombinant phage is separated from the SAK producing E. coli and digested with the same restriction enzyme(s) as was/were used for the digestion of the phage DNA and for the cleavage of the vector DNA. The molecular weights (i.e. the DNA lengths) of the resultant DNA fragments are measured by means known in the art, for example, by agarose gel electrophoresis, whereby it is possible to locate the SAK gene-carrying DNA fragment on the phage DNA.

An example of such analysis is shown below.

Analytical Example:

SφC DNA was employed as phage DNA. It was recognised to have genetic information encoded for the production of SAK:

(1) when digested with restriction enzyme Pst I, within the segment of 15.8 kb;

(2) when digested with restriction enzyme Hind III, within the segment of 4.9 kb;

(3) when digested with restriction enzymes Hind III and Ava II, within the segment of 2.0 kb; and

(4) when digested with restriction enzymes Ava II and Acc I, within the segment of 1.3 kb.

The SAK-producing E. coli obtained by the process described in the foregoing has the same microbiological characteristics as those of the original E. coli, except in that the former E. coli has the capability of production of SAK. Microbiological characteristics, determined with the E. coli strains obtained in Examples referred to later, are shown in the following table.

TABLE

Microbiological characteristics of E. coli strains imparted with capability
of producing SAK as well as E. coli host

|  | Strain | | |
| --- | --- | --- | --- |
|  | A* | B** | C*** |
| Gram's staining and morphology | negative bacillus | negative bacillus | negative bacillus |
| Catalase production | + | + | + |
| Oxidase production | − | − | − |
| OF test | F | F | F |
| Acid production from glucose | + | + | + |
| Gas generation from glucose | + | + | + |
| Nitrate reducibility | + | + | + |
| Phenylalanine deaminase activity | − | − | − |
| Lysine decarboxylase activity | − | − | − |
| Ornithine decarboxylase activity | − | − | − |
| Urease activity | − | − | − |
| Hydrogen sulfide production | − | − | − |
| Indole production | + | + | + |
| ONPG decomposability | − | − | − |
| DNA decomposition activity | − | − | − |
| Citrate assimilability | − | − | − |
| Maronate assimilability | − | − | − |
| Acid production from saccharides from lactose | − | − | − |
| from arabinose | + | + | + |
| from inositol | − | − | − |
| from rhamnose | + | + | + |

*A represents E. coli K12 C600 $r_K^- m_K^-$
**B represents E. coli K12 C600 $r_K^- m_K^-$ (pSAK-HP2)
***C represents E. coli K12 C600 $r_K^- m_K^-$ (pSAK 361)

The strain designated B above, which is included in the present invention, was deposited with the American Type Culture Collection of 12301 Parklawn Drive Rockville, Maryland 20852, U.S.A. on 23rd August 1982 under accession number ATCC 39179.

It will be appreciated that the present invention also provides an industrially advantageous process for the production of SAK which comprises culturing a novel E. coli in accordance with the invention (which may be obtained in the manner described previously) and collecting SAK accumulated in the cultured cells.

The SAK production process of the present invention is carried out by culturing in a manner conventional in the art, a new SAK producing E. coli strain of the invention, harvesting the cultured cells and then collecting the periplasmic fraction, from which most of the substance having SAK activity is obtained. The collection of the periplasmic fraction can be carried out by means and techniques known *per se* in the art (see, for example, Talmadge, K. et al. (1980) Proc. Natl. Acad. Sci. U.S.A. *77*, 3369—3373). The process of the invention is novel and effective, and different from the prior art process with S. aureus wherein the supernatant of the culture is collected. Thus, it has been rendered possible to recover SAK as product from the periplasmic space for the first time by the process of the invention, in which SAK can be produced by E. coli. This constituted one of the characteristic advantages of the process of the invention.

Purification of the thus-obtained SAK may be carried out by conventional means for the purification of proteins, for example, by salting out, gel filtration or adsorption chromatography.

Thus according to the present invention, which enables E. coli to produce SAK, many outstanding advantages are offered over the prior art using S. aureus, such as safety, growth rate and the feasibility of mass culture (i.e. mass production). Further outstanding advantages, which all stem from the fact that SAK accumulates in the periplasmic space, are that SAK is obtained in concentrations more than one hundred times higher than in the case of the prior art method using S. aureus in which SAK is obtained from a supernatant fraction, and that the isolation and purification of SAK can be carried out in an easier manner than by a method involving crushing of whole cells followed by extraction, as a result of the lower content of proteinaceous contaminants.

The above mentioned phenomenon of SAK accumulation in the periplasmic space can be demonstrated by the following. Thus, E. coli strain, E. coli K12 C600 $r_K^-m_K^-$ (pSAK 361), which was obtained in Example 1 described hereinafter, was cultured at 37°C for 5 hours in 200 ml of L broth. The distribution and relative amounts of SAK activity are shown below.

|  | Total activity (u) | % |
| --- | --- | --- |
| Supernatant fraction | 950 | 23 |
| Periplasmic fraction | 2400 | 58 |
| Cytoplasmic fraction | 800 | 19 |

This result shows that about 60% of SAK is present in the periplasmic fraction.

The following Examples serve to illustrate the present invention without limiting it.

Example I

In this Example SϕC was used as the DNA donor, pBR 322 as the vector DNA, Hind III as the restriction enzyme, and E. coli K12 C600 $r_K^-m_K^-$ or E. coli K12 WA802 $r_K^-m_K^-$ as the host E. coli strain.

(A) Preparation and Digestion of SϕC DNA

Phage SϕC was grown in accordance with method described in Blair, J.E. and Williams, R.E.O. (1961) Bull, W.H.O. *24*, 771—784. To 1 litre of the resulting phage lysate was added 100 ml of 5M-NaCl and then 300 ml of 30% (w/w) polyethylene glycol. The mixture was stirred well and allowed to stand at 0°C for several hours. The mixture was then subjected to centrifugation at 10,000 rpm for 15 minutes to obtain the precipitate. The precipitate was dissolved in 20 ml of buffer (pH 7.4) containing 10 mM Tris. HCl, 10 mM $MgSO_4$, 5 mM $CaCl_2$ and 0.01% gelatin. To the resulting solution were added DNase and RNase in a manner such that the final concentration of each enzyme became 1 μg/ml. The reaction was carried out at 37°C for 20 minutes. After the reaction, the mixture was centrifuged at 10,000 rpm for 10 minutes and the supernatant was further centrifuged at 30,000 rpm for 60 minutes. The thus deposited precipitate was dissolved in 2.5 ml of the same buffer as described above. About 1.7 g of cesium chloride was added to the solution and the mixture was subjected to equilibrium density gradient centrifugation at 27,000 rpm for 18 hours. The thus obtained band of the phage was separated and dialyzed against 1 litre of the same buffer as described above. To 0.5 ml of the dialyzed phage suspension were added 50 ul of 1.5 M NaCl—0.15 M sodium citrate, 5 μl of 250 mM disodium salt of EDTA (pH 7.0) and 5 μl of 10% SDS. After the mixture was allowed to stand at 37°C for 5 minutes, 0.6 ml of phenol saturated with 0.15 M NaCl—15 mM sodium citrate solution was added thereto and the resulting mixture was gently stirred at room temperature for about 10 minutes. The mixture was then subjected to centrifugation at 3,000 rpm for 10 minutes to collect the upper phase (i.e. aqueous phase). The aqueous phase was subjected to the phenol treatment described above and then washed three times with equi-volumes of ether. The resulting aqueous phase was dialyzed three times against 200 ml of 0.15 M NaCl—15 mM sodium citrate—1 mM disodium salt of EDTA.

Digestion of the thus obtained phage DNA was carried out as follows. Three units of Hind III were added to 1 μg of the DNA and the reaction was carried out at 37°C for 3 hours in 20 μl of a buffer containing 10 mM Tris. HCl—7 mM $MgCl_2$—7 mM β-mercaptoethanol—50 mM NaCl (pH 7.6). At the completion of the reaction 1 μl of 250 mM disodium salt of EDTA (pH 8.0) was added to the reaction mixture.

(B) Cleavage of plasmid pBR 322

One unit of Hind III was added to 1 μg of pBR 322 and the reaction was carried out at 37°C for 3 hours in 20 µl of the same buffer as used for the digestion of the phage DNA. At the completion of the reaction 1 µl of 250 mM disodium salt of EDTA (pH 8.0) was added to the reaction mixture.

(C) Ligation

Five µl of 1.5 M sodium acetate (pH 7.0) was added to 20 µl of each of the restriction enzyme-treated DNA solutions prepared in (A) and (B) above, respectively. Each mixture was supplemented with 75 µl of 95% ethanol, allowed to stand at −70°C for 10 minutes and subjected to centrifugation at 12,000 rpm for 5 minutes. Then each precipitate was collected, washed again with 95% ethanol, then dried under reduced pressure and dissolved in 10 µl of 10 mM tris · HCl—1 mM disodium salt of EDTA (pH 8.0; hereinafter referred to as TE buffer).

Ligation was carried out, either at 4°C for 48 hours or at 20°C for several hours, in 20 µl of a mixture containing the digested pBR 322 DNA (50 ng as pBR 322 DNA) which was obtained in (B), the cleaved SφC DNA (1.5 µg as SφC DNA) which was obtained in (A), 30 mM Tris-HCl (pH 7.6), 10 mM MgCl$_2$, 10 mM dithiothreitol, 0.1 mM ATP and 0.1 unit of T4 ligase. One µl of 0.25 M disodium salt of EDTA (pH 8.0) was added to the reaction mixture to terminate the reaction. The reaction mixture was heated at 65°C for 5 minutes to inactivate the ligase and stored at −20°C after addition of 180 µl of TE buffer.

(D) Introduction of recombinant plasmid into E. coli

Each E. coli strain was inoculated into 10 ml of L-broth (pH 7.0) consisting of 1% polypeptone, 0.5% yeast extract and 0.5% NaCl; and grown to $5 \times 10^8$ cells/ml. The mixture was centrifuged at 4°C to collect the cells. The cells were suspended in 5 ml of chilled 50 mM CaCl$_2$ and allowed to stand for 5 minutes in an ice water bath. The suspension was then centrifuged again to collect the cells. The cells are suspended in 0.67 ml of chilled 50 mM CaCl$_2$ and the suspension was allowed to stand for 5 minutes in an ince water bath. The E. coli suspension was mixed with 0.33 ml of a solution of the ligated plasmid DNA prepared in (C) above and the mixture was kept for 5 minutes in an ice water bath. The reaction mixture was held at 42°C for 2 minutes to terminate introduction of the recombinant plasmid into E. coli. The resulting suspension was, either as such or after being diluted ten to one hundred fold, plated on a L-broth agar plate containing 40 µg/ml of ampicillin. The agar plate was incubated overnight at 37°C to grow the E. coli transformants which had gained resistance to ampicillin.

(E) Selective separation of SAK producing E. coli

Five ml each of human plasma was charged into test tubes and heated at 56°C for 20 minutes. Separately, ten ml each of liquefied 2% agar-containing nutrient broth was charged into test tubes and kept warmed at 56°C. These two preparations were promptly mixed together in petri dishes and allowed to stand until the mixture soldified. Aliquots of sample suspensions of the E. coli cells obtained in (D) above were spotted on the thus prepared agar plate and incubated at 37°C. There were observed colonies around which transparent fibrinolytic halos put in an appearance. The appearance means that the microbe of the colonies have capability of SAK production.

The E. coli strains forming such colonies are novel E. coli strains according to the present invention. One of the obtained novel strains is designated as E. coli K12 C600 $r_K^- m_K^-$ (pSAK 361) and another one, as E. coli K12 WA802 $r_K^- m_K^+$ (pSAK 361), corresponding to the host E. coli strain, respectively.

E. coli K12 WA802 $r_K^- m_K^+$ (pSAK 361) was deposited on 23rd August 1982 with the American Type Culture Collection of 12301 Parklawn Drive, Rockville, Maryland 20852, U.S.A. under accession number ATCC 39178.

(F) Analysis of E. coli plasmid

The thus obtained E. coli cells of each strain were grown at 37°C in 400 ml of L-broth which contained 40 µg/ml of ampicillin but not glucose to about $4 \times 10^8$ cells/ml. At this point the culture was supplemented with chloramphenicol in a manner such that the latter's concentration became 150 µg/ml, and then subjected to further cultivation at 37°C for 15 hours followed by centrifugation to collect cells. The cells were washed in 50 ml of 10 mM Tris-HCl (pH 8.0)—0.015 M NaCl—1.5 mM sodium citrate and then suspended in 3.2 ml of 25% sucrose—50 mM Tris-HCl (pH 8.0). The suspension was supplemented with 1.6 ml of 250 mM disodium salt of EDTA (pH 8.0), 1 ml of 5 mg/ml lysozyme and 6 ml of 2% Brij-58 (KAO-ATLAS Co., Ltd.)—62.5 mM disodium salt of EDTA—50 mM Tris · HCl (pH 8.0) and incubated at 30°C for 15 minutes to lyse mildly the cell walls. The mixture was subjected to high speed (30,000 rpm) centrifugation at 4°C for 30 minutes to precipitate the cells. The supernatant was subjected to CsCl-ethidium bromide equilibrium density gradient centrifugation (36,000 rpm) at 10°C for 48 hours to purify the plasmid DNA. The resulting plasmid DNA was digested with Hind III in accordance with the same procedure as described in (B) above. Length of the SAK gene-carrying DNA was determined to be 4.9 kb upon measurement by agarose gel electrophoresis using 1% agarose.

(G) Separation of 4.9 kb fragment

One hundred µg of SφC was digested by reacting the same, at 37°C for 20 hours, with a mixture containing 10 mM Tris-HCl (pH 7.6), 7 mM MgCl$_2$, 7 mM β-mercaptoethanol, 50 mM NaCl and 100 units Hind III. The reaction mixture was subjected to agarose gel electrophesis using 1% agarose. DNA was stained with ethidium bromide and only the desired region of the gel, i.e. the region containing the 4.9 kb DNA fragment, was cut out. The 4.9 kb fragment was eluted from an agarose gel electrophoretically into a dialysis tubing. The DNA eluate was treated with TE buffer-saturated phenol and the aqueous phase was treated first with ether and then with ethanol to precipitate DNA. The resulting DNA is ready for use in ligation with vector DNAs.

(H) Production, extraction and purification of SAK

The SAK producing E. coli cells obtained above were inoculated into 50 ml of L-broth containing 1% polypeptone, 0.5% yeast extract, 0.5% NaCl and 0.1% glucose (pH 7.0), and subjected, overnight at 37°C, to shaking culture. The resulting culture was inoculated into 5 litres of the same broth as used above, and subjected to shaking culture at 37°C for 12 hours. The culture was then centrifuged to collect cells. The cells were suspended in 200 ml of 100 mM Tris-HCl—20% sucrose (pH 8.0) and the suspension was centrifuged again to collect cells. The cells were resuspended in 45 ml of 100 mM Tris-HCl—20% sucrose (pH 8.0) and the suspension was supplemented with 5 ml of a lysozyme solution (5 mg/ml lysozyme in 20 mM disodium salt of EDTA, pH 8.0) and then allowed to stand in an ice water bath for 1 hour. The mixture was centrifuged to collect the supernatant. To the supernatant was added ammonium sulfate to 80% saturation. The mixture was allowed to stand overnight at 4°C and then centrifuged to collect the precipitate. The precipitate was dissolved in 10 ml of 10 mM Tris-HCl (pH 7.5) and dialyzed against the same buffer. The dialysate was subjected to Sephadex G-75 gel chromatography and eluted with 10 mM Tris-HCl (pH 7.5) to collect fractions of SAK activity. The fractions so collected were adsorbed to a column of CM-cellulose equilibrated with 10 mM Tris-HCl (pH 7.5), and subjected to linear gradient elution with 0—0.5 M NaCl. Fractions of SAK activity were collected which were eluted with 0.3—0.32 M NaCl.

Example II

In this Example, S$\phi$C was used as the DNA donor, Hind III and PstI as the restriction enzymes for digesting S$\phi$C, pBR 322 as the vector DNA, EcoRI and PstI as the restriction enzymes for digesting pBR 322, and E. coli K12 C600 $r_K^- m_K^-$ and E. coli K12 WA802 $r_K^- m_K^+$ as the E. coli strains.

(A) Digestion of phage S$\phi$C DNA

Following the procedures described in Example I A through F, a 4.9 kb fragment obtained by digesting the S$\phi$C DNA with Hind III was isolated. The single-stranded portions, present at both termini of the fragment and each consisting four bases, were modified into double-strands by reacting the fragment at 18°C for 4 hours with 1 unit of T4 DNA polymerase and four kinds of deoxyribonucleoside triphosphates (25 $\mu$M each) in 20 $\mu$l of a buffer containing 67 mM Tris-HCl (pH 8.0), 6.7 mM $MgCl_2$ and 6.7 mM $\beta$-mercaptoethanol. To the reaction mixture were added 5 $\mu$l of 1.5 M sodium acetate (pH 7.0) and 75 $\mu$l of ethanol. The mixture was held at −70°C for 10 minutes and then subjected to centrifugation at 12,000 rpm for 5 minutes. The precipitate was washed with ethanol and dissolved in a buffer containing 10 mM Tris-HCl (pH 7.6)—7 mM $MgCl_2$—7 mM $\beta$-mercaptoethanol—50 mM NaCl. To the solution was added 0.5 unit of PstI and the reaction was carried out at 37°C for 3 hours to further digest the fragment into two segments, which were ready for use in ligation in (C) below.

(B) Cleavage of Plasmid pBR 322

One unit of EcoRI was added to 1 $\mu$g of pBR 322 DNA and the reaction was carried out in the same manner as in Example I (B) to effect the cleavage. The single-stranded portions, present at both termini of the cleaved DNA and consisting of four bases, were modified into double strands in the same manner as in (A) above using T4 DNA polymerase and four kinds of deoxyribonucleoside triphosphates. The resulting entirely double-stranded DNA was digested with PstI in the same manner as in (A) above. The digests were ready for use in ligation in (C) below.

(C) Ligation

The DNA fragments from S$\phi$C DNA and plasmid pBR 322 were mixed together and subjected to ligation in the same manner as in Example I (C), except that the amount of T4 DNA ligase used was 1 unit.

(D) Subsequently, the same procedures as in Example I were followed to obtain two novel E. coli strains, which were designated E. coli K12 C600 $r_K^- m_K^-$ (pSAK-HP2) and E. coli K12 WA802 $r_K^- m_K^+$ (pSAK-HP2). Plasmid analysis revealed that the DNA of each of the E. coli strains had S$\phi$C-derived genetic information for SAK production of about 2.5 kb, in terms of DNA length, inserted.

Example III

In this Example, P$\phi$1 was used as the DNA donor, pBR 322 as the vector, Hind III as the restriction enzyme and E. coli K12 C600 $r_K^- m_K^-$ as the E. coli strain.

(A) Preparation, and digestion with Hind III, of phage P$\phi$1 DNA, as well as Digestion of vector DNA, were carried out in the same manner as in Example I.

(B) Ligation reaction, introduced into E. coli, and selection of recombinant E. coli cells were carried out in the same manner as in Example I.

# EP 0 077 664 B1

(C) As a result of these operations, the DNA segment carrying genetic information for SAK production which segment derived from Pφ1 was successfully introduced into the E. coli strain. The resulting novel E. coli strain was designated as E. coli K12 C600 $r_K^- m_K^-$ (pSAK 601).

## Claims

1. Recombinant DNA carrying genetic material encoded for the production of staphylokinase and derived from a temperate phage of Staphylococcus aureus, said DNA being obtainable from ATCC 39179 or ATCC 39178 in the form of a sequence 4.9 kb in size by digestion with Hind III, or said DNA being DNA exhibiting at least partial homology to said sequence, in either case the DNA being capable of transforming a host coliform bacillus to introduce said genetic material into the host genotype whereby said genetic material may be phenotypically expressed by the host to produce staphylokinase.

2. Recombinant DNA which is pSAK-HP2, (obtainable from ATCC No. 39179) or pSAK361 (obtainable from ATCC No. 39178).

3. A coliform bacillus having as part of its phenotypically expressible genotype recombinant DNA as claimed in claim 1 or claim 2.

4. A bacillus which is a strain of E. coli K12 C600 $r_K^- m_K^-$ or of E. coli K12 WA802 $r_K^- m_K^+$ and in either case incorporating in its genome recombinant DNA as claimed in Claim 2.

5. A process for preparing a bacillus as defined in claim 3, which process comprises subjecting a temperate phage DNA of Staphylococcus aureus to restriction enzyme digestion to produce DNA fragments, optionally isolating the DNA fragment which is encoded for staphylokinase production, cleaving a DNA vector capable of transforming a host coliform bacillus with one or more restriction enzymes, ligating said fragment or fragments into resulting vector cleavage site(s), introducing the resulting recombinant DNA into a coliform bacillus host, and selectively separating those of the resulting coliform bacillus cells which have thereby gained the capability of producing staphylokinase.

6. A process for producing staphylokinase comprising cultivating a coliform bacillus as claimed in claim 3 or claim 4 to accumulate staphylokinase and collecting the thus-produced staphylokinase.

## Patentansprüche

1. Rekombinante DNA, die genetisches Material trägt, das für die Herstellung von Staphylokinase kodiert und von einem temperenten Phagen von Staphylococcus aureus hergeleitet ist, wobei diese DNA aus ATCC 39179 oder ATCC 39178 in Form einer Sequenz von 4,9 kb Länge durch Verdauung mit Hind III erhältlich ist, oder diese DNA eine DNA darstellt, die eine wenigstens teilweise Homologie zu dieser Sequenz aufweist, wobei die DNA in jedem dieser Fälle geeignet ist, einen coliähnlichen Wirtsbazillus zu transformieren um das besagte genetische Material in das Wirtsgenom einzuführen, wodurch das genetische Material vom Wirt phenotypisch unter Erhalt von Staphylokinase exprimiert werden kann.

2. Rekombinante DNA, nämlich pSAK-HP2 (erhältlich aus ATCC 39179) oder pSAK361 (erhältlich aus ATCC 39178).

3. Coliähnlicher Bazillus, der als Teil seines phenotypisch exprimierbaren Genomtyps rekombinante DNA nach den Ansprüchen 1 oder 2 enthält.

4. Bazillus vom Stamm E. coli K12 C600 $r_K^- m_K^-$ oder E. coli K12 WA802 $r_K^- m_K^+$, der in jedem Fall in sein Genom rekombinante DNA nach Anspruch 2 inkorporiert.

5. Verfahren zur Herstellung eines Bazillus gemäss Anspruch 3, wobei das Verfahren das Spalten einer DNA eines temperenten Phagen von Staphylococcus aureus mit Restriktionsenzymen unter Erhalt von DNA-Fragmenten, gewünschtenfalls das Isolieren des für die Staphylokinaseherstellung kodierenden DNA-Fragmentes, Schneiden eines DNA-Vektors, der in der Lage ist, einen coliähnlichen Wirtsbazillus zu transformieren, mit einem oder mehreren Restriktionsenzymen, Ligation dieser (dieses) Fragmente(s) in die entstehende(n) Vektorschnittstelle(n), Einführen der erhaltenen rekombinanten DNA in einen coliähnlichen Wirtsbazillus, und das selektive Abtrennen jener entstehenden coliähnlichen Bazilluszellen, die solchermassen die Fähigkeit zur Herstellung von Staphylokinase erworben haben, umfasst.

6. Verfahren zur Herstellung von Staphylokinase, umfassend das Kultivieren eines coliähnlichen Bazillus nach den Ansprüchen 3 oder 4, wobei Staphylokinase angesammelt wird, und das Sammeln der so hergestellten Staphylokinase.

## Revendications

1. ADN recombinant portant un matériel génétique codé pour la production de staphylokinase et dérivé d'un phage tempéré de Staphylococcus aureus, ledit ADN pouvant être obtenu à partir d'ATCC 39179 ou d'ATCC 39178 sous forme d'une séquence ayant une taille de 4,9 kb par digestion avec Hind III, ou ledit ADN étant un ADN présentant au moins une homologie partielle avec ladite séquence, dans les deux cas l'ADN étant capable de transformer un bacille coliforme hôte pour introduire ledit matériel génétique dans le génotype de l'hôte, si bien que ledit matériel génétique peut être exprimé phénotypiquement par l'hôte pour produire la staphylokinase.

2. ADN recombinant qui est pSAK-HP2 (pouvant être obtenu à partir d'ATCC n° 39179) ou pSAK 361 (pouvant être obtenu à partir d'ATCC n° 39178).

3. Bacille coliforme ayant comme partie de son génotype expressible phénotypiquement de l'ADN recombinant comme revendiqué dans la revendication 1 ou dans la revendication 2.

4. Bacille qui est une souche d'E. coli K12 C600 $r_K^- m_K^-$ ou de E. coli K12 WA802 $r_K^- m_K^+$ et au génome duquel, dans les deux cas, est incorporé l'ADN recombinant comme revendiqué dans la revendication 2.

5. Procédé pour préparer un bacille comme défini dans la revendication 3, lequel procédé consiste à soumettre un ADN de phage tempéré de Staphylococcus aureus à une digestion avec une enzyme de restriction pour produire des fragments d'ADN, facultativement isoler le fragment d'ADN qui est codé pour la production de staphylokinase, cliver un ADN vecteur capable de transformer un bacille coliforme hôte avec une ou plusieurs enzymes de restriction, ligaturer ledit fragment ou lesdits fragments au(x) site(s) de clivage du vecteur obtenu, introduire l'ADN recombinant obtenu dans un bacille coliforme hôte et séparer sélectivement les cellules de bacille coliforme obtenues qui ont ainsi acquis la capacité de produire la staphylokinase.

6. Procédé pour la production de staphylokinase comprenant la culture d'un bacille coliforme comme revendiqué dans la revendication 3 ou la revendication 4 pour accumuler de la staphylokinase et récolter la staphylokinase ainsi produite.